(19) Europäisches Patentamt
European Patent Office
Office européen des brevets

(11) Publication number: **0 253 472 B1**

(12) # EUROPEAN PATENT SPECIFICATION

(45) Date of publication of patent specification: **06.05.92**

(51) Int. Cl.5: **A61K 31/19**, A61K 31/215, A61K 31/365, A61K 47/00

(21) Application number: **87304334.3**

(22) Date of filing: **15.05.87**

(54) **Ophthalmic flurbiprofen preparation.**

(30) Priority: **16.05.86 JP 113383/86**

(43) Date of publication of application:
**20.01.88 Bulletin 88/03**

(45) Publication of the grant of the patent:
**06.05.92 Bulletin 92/19**

(84) Designated Contracting States:
**BE DE FR GB NL SE**

(56) References cited:
**EP-A- 0 082 921**
**EP-A- 0 129 435**

**PATENT ABSTRACTS OF JAPAN, vol. 8 no. 118 (C-226) (1555), 31 th May 1984; & JP-A-59 29616 (KAKEN SEIYAKU K.K.) 16-02-1984.**

**PATENT ABSTRACTS OF JAPAN, vol. 6, no. 191 (C-127) (1069), 30th September 1982; & JP-A-57 102 817 (KAKEN YAKUKAKOU K.K.) 26-06-1982.**

(73) Proprietor: **GREEN CROSS CORPORATION**
**15-1, Imabashi-1-chome Higashi-ku Osaka-shi**
**Osaka 541(JP)**

Proprietor: **KAKEN PHARMACEUTICAL CO., LTD.**
**No. 28-8, 2-chome, Honkomagome Bunkyo-Ku**
**Tokyo 113(JP)**

(72) Inventor: **Mizushima, Yutaka**
**25-20, Daita-4-chome Setagaya-ku**
**Tokyo(JP)**
Inventor: **Okamoto, Hiroyuki**
**3-18-302 Nagatecho-5-chome Nada-ku**
**Kobe(JP)**
Inventor: **Sugio, Shigetoshi**
**Midori Juji Ryokufuryo, 24-9 Kitanakaburi-1-chome**
**Hirakata-shi(JP)**
Inventor: **Yokoyama, Kazumasa**
**7-2-201, Terauchi-2-chome**
**Toyonaka-shi(JP)**
Inventor: **Suyama, Tadakazu**
**3-7, Matsuigaoka-4-chome Tanabecho**
**Tsuzuki-gun Kyoto(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 253 472 B1

Rank Xerox (UK) Business Services
(3.08/2.18/2.0)

Inventor: **Tono,Masao**
**2-4, Tsukinowa-3-chome**
**Otsu-shi(JP)**
Inventor: **Okumura, Makoto**
**330, Imajukucho-2-chome**
**Moriyama-shi(JP)**
Inventor: **Konishi, Yoshiaki**
**23, Koaza Ayasugigawara Oaza Hirao**
**Yamashirocho**
**Soraku-gun Kyoto(JP)**
Inventor: **Ichikawa, Kiyonoshin**
**78, Yagicho-9-chome**
**Omihachiman-shi(JP)**
Inventor: **Uchida, Katsuhiro**
**336, Kashiwayacho Yanaginobanbadori-**
**Gojoagaru**
**Shimogyo-ku Kyoto(JP)**

(74) Representative: **Ellis, Edward Lovell et al**
**MEWBURN ELLIS & CO. 2/3 Cursitor Street**
**London EC4A 1BO(GB)**

## Description

This invention relates to an ophthalmic preparation containing a substance active as flurbiprofen (hereinafter sometimes referred to as "FP").

Flurbiprofen [chemical name: 2-(2-fluoro-4-biphenylyl) propionic acid] has superior antiinflammatory, analgesic and antipyretic activities and has been sold in the form of tablets. Esters thereof have been included in an oil-in-water (a so-called "fat") emulsion (EP-A-0129435).

However, FP is sparingly soluble in water, and hence it has not been possible, to date, to incorporate FP as such into ophthalmic medicaments such as ophthalmic lotions for treating inflammation of the eyes.

Methods of forming derivatives of FP which can be formulated as ophthalmic lotions are known, namely a method of formation of a salt of FP(JP-A-102817/82) and a method of forming a clathrate of FP with cyclodextrin (EP-A-0082921).

It is desirable to provide a formulation whereby FP itself or a derivative thereof which is sparingly soluble in water can be administered as an ophthalmic preparation such as an ophthalmic lotion to allow treatment of inflammation of the eyes.

For an ophthalmic preparation containing FP or a derivative thereof, it is important that the FP or its derivative can migrate sufficiently to the anterior chamber of the eye.

Furthermore, solubility, stability and stimulation are important for ophthalmic preparations, especially ophthalmic lotions.

Accordingly, an object of this invention is to provide an ophthalmic preparation containing FP or a derivative thereof which effectively migrates to the anterior chamber and besides is excellent in solubility in aqueous solvents, storage stability and non-stimulation of the eye.

The inventors have made intensive researches in an attempt to solve the above problems and found that the above object can be attained by using a fat emulsion of FP or derivative thereof as an ophthalmic preparation.

That is, this invention relates to an ophthalmic preparation comprising a fat emulsion containing a phospholipid emulsifier and emulsified soybean oil in which, as an active ingredient, flurbiprofen or a derivative thereof is dissolved, the derivative being a soybean oil-soluble ester of flurbiprofen; characterized in that the fat emulsion is adapted for only topical, to the exclusion of oral, administration as an ophthalmic medicine preparation.

The FP derivatives used in this invention have no special limitation as long as they have an activity as FP and can be soluble in soybean oil and emulsified into a fat emulsion and some of the derivatives as well as fat emulsion thereof are known, for example, from JP-A-13720/84 and US-A-4613505, neither of which mentions ophthalmic preparations containing the fat emulsions.

One of the derivatives is an ester of FP and is disclosed in US-A-4613505.

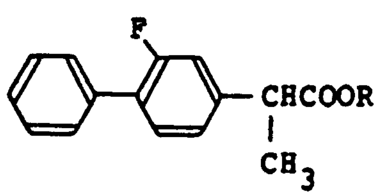

(1)

wherein R is an alkylcarbonyloxyalkyl group or an alkenylcarbonyloxyalkyl group represented by the formula (A)

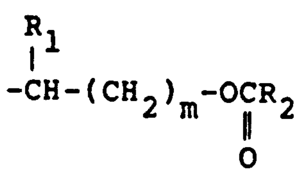

(A)

wherein $R_1$ is hydrogen atom or a $C_1$-$C_3$ alkyl group, $R_2$ is a $C_1$-$C_{15}$ alkyl group or a $C_2$-$C_8$ alkenyl group, and m is zero or an integer of 1; or a lactone group represented by the formula (B)

$$-CHCOO(CH_2)_n-C(R_3)(R_4) \qquad (B)$$

wherein $R_3$ and $R_4$ are same as or different from each other and are a hydrogen atom or a $C_1$-$C_3$ alkyl group, and n is an integer of 1 or 2.

Typical examples of the group represented by R in the general formula (I), which fall in the formula (A), include acetoxymethyl group, propionyloxymethyl group, isobutyryloxymethyl group, pivaloyloxymethyl group, palmitoyloxymethyl group, crotonoyloxymethyl group, 3,3-dimethylacryloyloxymethyl group, 1-acetoxyethyl group, 1-acetoxypropyl group, 1-propionyloxyethyl group, 1-isobutyryloxyethyl group, 1-pivaloyloxyethyl group, 1-palmitoyloxyethyl group, 1-crotonoyloxyethyl group, 1-(3,3-dimethylacryloyloxy)-ethyl group, 1-(2,4-hexadienoyloxy)ethyl group, 2-acetoxyethyl group, 2-propionyloxyethyl group, 2-crotonoyloxyethyl group, 2-(3,3-dimethylacryloyloxy)ethyl group, 2-(2,4-hexadienoyloxy)-ethyl group and 2-(3,7-dimethyl-2,6-octadienoyloxy)ethyl group, and the lactone groups which fall in the formula (B) are 3,3-dimethyl-γ-butyrolacton-2-yl group, 3,3-dimethyl-γ-valerolacton-2-yl group and 3-methyl-3-propyl-γ-butyrolacton-2-yl group.

Of the esters defined in the above formula, a lower alkanoyloxy-lower alkyl ester such as l-acetoxyethyl (or methyl) 2-(2-fluoro-4-biphenyl)propionate is preferable.

Another of the derivatives suitably used in the invention is an alkyl ester of FP as is disclosed in JP-A-13720/84. The alkyl of the ester is selected from $C_1$-$C_{18}$ alkyls which may be of straight or branched form.

Compositions of the fat emulsion suitable for use in this invention comprise generally 0.001 - 5% (W/V), preferably 0.01 - 1% (W/V) of FP or its derivative, 0.1 - 50% (W/V), preferably 1 - 20% (W/V) of soybean oil, 1 - 50 parts, preferably 5 - 30 parts of phospholipid based on 100 parts by weight of the soybean oil and a suitable amount of an aqueous solvent, the FP or its derivative being dissolved in soybean oil emulsified.

As soybean oil, there may be generally used a purified soybean oil of high purity, which is disclosed, for example, in Journ. Am. Oil Chemist Soc. 27 422 - 423. Furthermore, as the phospholipid, there may also be generally used a purified phospholipid or a hydrogenated phospholipid which is derived from soybean or egg yolk, as disclosed in Journ. Biol. Chem. 192 723 - 628 (1951).

There may be used any aqueous solvents which are physiologically acceptable, for example, distilled water for injection, physiological saline solution, phosphate buffer solution, borate buffer solution, or citrate buffer solution.

The fat emulsion of this invention may further contain up to 0.3% (W/V) of a fatty acid having 6 - 22, preferably 12 - 20 carbon atoms or a physiologically acceptable salt thereof as an emusion adjuvant. These fatty acids and salts thereof may be known ones. The emulsion may also contain 0.5% (W/V) or less, preferably 0.1% or less of a cholesterol or 5% (W/V) or less, preferably 1% (W/V) or less of phosphatidic acid as a stabilizer.

Furthermore, the preparation of this invention may also contain as a stabilizer a high molecular weight substance selected from albumin, dextran, vinyl polymer such as a polyvinyl pyrrolidone, nonionic surface active agent such as a polyalkylene glycol of a molecular weight of 1,000 to 10,000 and a polyoxyethylene-polyoxypropylene copolymer of a molecular weight of 1,000 to 20,000, gelatin and hydroxyethyl starch. The amount of the stabilizer added may he 0.1-5 parts by weight, preferably 0.5 - 1 part by weight based on one part by weight of FP or its derivative. The preparation of the fat emulsion is concretely disclosed, for example, in US-A-4 613 505.

The fat emulsion of this invention may be prepared by using an ordinary homogenizer, for example, a pressure-jet type homogenizer or an ultrasonic homogenizer. That is, predetermined amounts of soybean oil, a phospholipid, FP or its derivative and, if necessary, other additives as mentioned above are mixed and the mixture is heated to form a solution, which is subjected to homogenizing treatment to form a water-in-oil type dispersion. A required amount of water is added to this dispersion and this is again subjected to homogenizing treatment by the homogenizer to convert the dispersion into an oil-in-water type emulsion. The stabilizer may be added to thus obtained emulsion.

The average particle size of the fat emulsion in a preparation of this invention is preferably 1.0 μm or less (particle size distribution 0.1 - 1.0 μm).

Specific embodiments of the preparation of this invention are, for example, ophthalmic lotions and ointments.

As ophthalmic lotions, the fat emulsion obtained as above may be used as it is in the form of a liquid preparation or may be formulated by freeze-drying by a conventional method. The freeze-dried preparation is generally diluted with or dispersed in a physiological aqueous solution for use.

The ophthalmic lotion is preferably isotonic. For isotonification, it is also possible to add a conventional isotonifying agent such as glycerine, glucose or a salt (e.g., boric acid or sodium chloride). The isotonifying agent may be added during the preparation of the fat emulsion.

It is also possible as a matter of course to use additives added in the preparation of ordinary ophthalmic lotions (preservatives such as methyl paraoxybenzoic acid, propyl paraoxybenzoic acid, sodium dehydroacetate and thimerosal; thickening agents such as polyvinyl pyrrolidone and sodium polyacrylate).

The pH of the ophthalmic lotion is usually about 5 - 9, preferably about 6 - 8.

The ophthalmic lotion may be preserved at room temperature even in a diluted form as if an aqueous solution, but preferably is preserved at a lower temperature of about 4°C. The ophthalmic lotion is administered in the dosage form of a dropping lotion or eye perfusion.

Ophthalmic ointment is prepared, for example, by mixing and dispersing freeze-dried powders of the fat emulsion with an ointment base.

The ointment base may be any of the known ones and may be either an aqueous or oily base. Typical examples thereof are vaseline and polyethylene glycol.

The preparation of this invention may be sterilized by any known method, for example, heat-steam sterilization or filtration sterilization.

The dose of the emulsion preparation of this invention varies depending on sex, age, symptoms of patients, but generally is administered at a dose of 0.04 - 2.0 ml/day in terms of fat emulsion and 0.1 - 1000 $\mu$g/day in terms of FP in about 1 - 10 times.

When the emulsion preparation of this invention is applied, FP or its derivative contained therein effectively migrates into the anterior chamber and thus the preparation is effective for treatment of inflammation of the conjunctiva and corneal epithelium.

Furthermore, the preparation has little toxicity and gives substantially no stimulus to the eye and besides is excellent in stability in an aqueous solvent and storage stability. Therefore, it is markedly superior as an ophthalmic preparation, especially an ophthalmic lotion.

The following nonlimiting examples and test examples further illustrate this invention.

Example 1

To 200.0 g of purified soybean oil were added 24.0 g of purified egg yolk phospholipid, 2 g of flurbiprofen, 0.5 g of sodium oleate and 0.5 g of phosphatidic acid and the mixture was heated at 40 - 75°C to form a solution. To this solution was added 1000 ml of distilled water. This mixture was emulsified by passing it through a Manton-Gaulin type homogenizer under pressure of 98,1 bar (100 kg/cm$^2$) in the first stage and 10 times under total pressure of 441,5 bar (450 kg/cm$^2$). Then, to this emulsion was added 44.2 g of glycerine and was further added 730 ml of distilled water for injection of 20 - 40°C and then, the mixture was treated with a homogenizer to obtain a crude emulsion. This crude emulsion was again emulsified by passing it through a Manton-Gaulin type homogenizer under pressure of 117,7 bar (120 kg/cm$^2$) in the first stage and 10 times under total pressure of 490,50 bar (500 kg/cm$^2$). Thus, a homogenized extremely fine fat emulsion containing flurbiprofen (pH 7) was obtained.

Example 2

To 20 g of purified soybean oil was added 0.4 g of flurbiprofen cetyl ester to form a solution at 80°C. Then, to the solution was added 5 g of purified egg yolk phospholipid and this mixture was vigorously stirred at 80°C to form a solution, followed by adding 200 ml of distilled water and stirring by homomixer to form a crude emulsion. This crude emulsion was emulsified as in Example 1 under high pressure by a Manton-Gaulin type homogenizer to obtain an extremely fine fat emulsion (pH 7) containing flurbiprofen cetyl ester.

Example 3

To 30 g of purified soybean oil were added 3.6 g of a phospholipid, 3 mg of acetoxymethyl 2-(2-fluoro-4-biphenyl)propionate,0.15 g of sodium oleate and 0.15 g phosphatidic acid and the mixture was heated at 40 - 75 ° C to for a solution. The solution was mixed with 200 ml of distilled water and then 7.5 of glycerol (Japanese Pharmacopoeia), made up into a total volume of 300 ml with distilled water for injection of 20 - 40 ° C and then treated with a homomixer to form a crude emulsion.

The crude emulsion was then emulsified by passing it through a Manton-Gaulin type homogenizer under pressure of 117,7 bar (120 kg/$cm^2$) in the first stage and 10 times under total pressure of 490,5 bar (500 kg/$cm^2$). Thus, a homogenized, extremely fine fat emulsion (pH 7) was obtained. The emulsion had an average particle diameter of 0.2 - 0.4 μm and contained no particles larger than 1 μm.

Example 4

Example 3 was repeated except that 1-acetoxyethyl 2-(2-fluoro-4-biphenylyl)propionate was used in place of acetoxymethyl 2-(2-fluoro-4-biphenylyl)-propionate. As a result, a homogenized, extremely fine fat emulsion (pH 7) was obtained. The emulsion had an average particle diameter of 0.2 - 0.4 μm and contained no particles larger than 1 μm.

Example 5

In the same manner as in Example 1 a fat emulsion was obtained from the following compositions. This emulsion had a pH of 7.5.

| | |
|---|---|
| 1-Acetoxyethyl 2-(2-fluoro-4-biphenylyl)propionate | 0.1% (W/V) |
| Soybean oil | 10% W/V) |
| Egg yolk phospholipid | 1.2% (W/V) |
| Glycerol | 2.21% (W/V) |
| Disodium hydrogen phosphate | 0.0284% (W/V) |
| Citric acid | 0.0961% (W/V) |
| Purified water | balance amount |

Test Example 1 (Toxicity in application of ophthalmic lotion)

The fat emulsion of the FP derivative obtained in Example 4 was tested for toxicity on application of the emulsion as an ophthalmic lotion using white rabbits in accordance with the Draise method (Jour. Pharmacol. Exp. Ther. 83 377-390 (1944). Physiological saline solution was used as a control and each of FP-sodium salt and FP-cyclo-dextrin solutions were used as control chemicals. As a result, it has been found that toxicity of the fat emulsion is similar to that of the physiological saline solution and is much lower than those of the control chemicals.

Test Example 2

50 μl of each of the following ophthalmic lotions as shown below [containing 0.1% (W/V) in terms of FP] was dropped in the eyes of white rabbits and after one hour, aqueous humor was collected and the concentration of FP in the aqueous humor was measured by HPLC. The results are shown in Table 1. The results indicate that the FP sufficiently migrates into the anterior chamber and antiinflammation and analgesic effects can be expected.

Table 1

| Ophthalmic lotion | Concentration of PF (μg/ml) |
|---|---|
| FP fat emulsion (Example 1)<br>Derivative emulsion (Example 4) | 1.0<br>1.3 |

## Claims

**1.** A fat emulsion containing a phospholipid emulsifier and emulsified soybean oil in which, as an active ingredient, flurbiprofen or a derivative thereof is dissolved, the derivative being a soybean oil-soluble ester of flurbiprofen; characterised in that the fat emulsion is adapted for only topical, to the exclusion of oral, administration as an ophthalmic medicine preparation.

**2.** A fat emulsion according to claim 1, wherein the fat is emulsified in an aqueous medium.

**3.** A fat emulsion according to claim 2, which contains 0.001 - 5% (W/V) of flurbiprofen, 1 - 20% (W/V) of soybean oil, 1 - 50 parts by weight of phospholipid based on 100 parts by weight of the soybean oil and as the balance an aqueous medium.

**4.** A fat emulsion according to any preceding claim, which additionally includes, as an emulsifying adjuvant, a fatty acid having 6 to 22 carbon atoms or a physiologically acceptable salt thereof.

**5.** A fat emulsion according to any preceding claim, which additionally includes, as a stabilizer, cholesterol, phosphatidic acid or a mixture thereof.

**6.** A fat emulsion according to any preceding claim, which additionally includes, as a stabilizer, an albumin, a dextran, a polyvinyl pyrrolidone, a polyalkylene glycol having a molecular weight of 1,000 to 10,000, a polyoxyethylene-polyoxypropylene copolymer having a molecular weight of 1,000 to 20,000, gelatin or hydroxyethylstarch.

**7.** A fat emulsion according to any preceding claim, which is freeze-dried.

**8.** A fat emulsion according to any preceding claim, which is in the form of an ophthalmic lotion.

**9.** A fat emulsion according to any one of claims 1 to 6, which is in the form of an ophthalmic ointment.

**10.** A fat emulsion according to claim 9, wherein the ointment comprises a freeze-dried powder of the fat emulsion and an ointment base.

**11.** A fat emulsion according to any preceding claim, wherein the active ingredient is flurbiprofen.

**12.** A fat emulsion according to any one of claims 1 to 10, wherein the active ingredient is a derivative of flurbiprofen, which derivatve is an ester of flurbiprofen represented by the formula

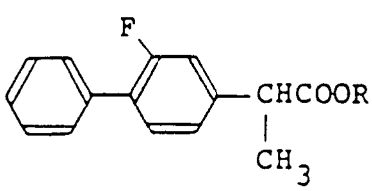

wherein R is a $C_1$-$C_{18}$ alkyl group or a group of the formula

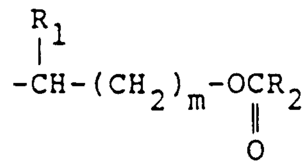

wherein $R_1$ is a hydrogen atom or a $C_1$ -$C_3$ alkyl group, $R_2$ is a $C_1$-$C_{15}$ alkyl group or a $C_2$-$C_8$ alkenyl group, and m is zero or 1; or a lactone group of the formula

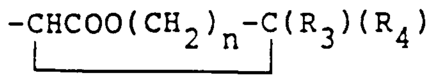

wherein $R_3$ and $R_4$ are the same as or different from each other and are each a hydrogen atom or a $C_1$-$C_3$ alkyl group, and n is 1 or 2.

**13.** A fat emulsion according to claim 12, wherein the flurbiprofen derivative is flurbiprofen cetyl ester, flurbiprofen acetoxymethyl ester or flurbiprofen acetoxyethyl ester.

**14.** The use of a fat emulsion in the manufacture of an ophthalmic medicament for topical treatment of inflammation of the eye, which fat emulsion contains a phospholipid emulsifier, an emulsified soybean oil and flurbiprofen, or a soybean oil-soluble derivative thereof, dissolved in the soybean oil.

**15.** The use according to claim 14, wherein the fat emulsion is as defined in any one of claims 1 to 13.

**Revendications**

**1.** Emulsion grasse contenant un émulsifiant phospholipidique et de l'huile de soja émulsionnée dans laquelle, comme ingrédient actif, du flurbiprofène ou un dérivé de celui-ci est dissous, le dérivé étant un ester de flurbiprofène soluble dans l'huile de soja; caractérisée en ce que l'émulsion grasse est adaptée seulement à une administration topique, à l'exclusion d'une administration orale, comme une préparation médicamenteuse ophtalmologique.

**2.** Emulsion grasse selon la revendication 1, dans laquelle la graisse est émulsionnée dans un milieu aqueux.

**3.** Emulsion grasse selon la revendication 2, qui contient 0,001-5% (P/V) deflurbiprofène, 1-20% (P/V) d'huile de soja, 1-50 parties en poids de phospholipide basé sur 100 parties en poids de l'huile de soja et comme reliquat un milieu aqueux.

**4.** Emulsion grasse selon une quelconque revendication précédente, qui inclut additionnellement, comme adjuvant émulsifiant, un acide gras ayant 6 à 22 atomes de carbone ou un sel physiologiquement acceptable de celui-ci.

**5.** Emulsion grasse selon une quelconque revendication précédente, qui inclut additionnellement, comme stabilisateur, du cholestérol, de l'acide phosphatidique ou un mélange de ceux-ci.

**6.** Emulsion grasse selon une quelconque revendication précédente, qui inclut additionnellement, comme stabilisateur, une albumine, un dextrane, une polyvinyl-pyrrolidone, un polyalkylèneglycol ayant une masse moléculaire de 1.000 à 10.000, un copolymère polyoxyéthylène-polyoxypropylène ayant une masse moléculaire de 1.000 à 20.000, de la gélatine ou de l'amidon d'hydroxyéthyle.

**7.** Emulsion grasse selon une quelconque revendication précédente, qui est séchée par congélation.

**8.** Emulsion grasse selon une quelconque revendication précédente, qui est sous la forme d'une lotion ophtalmologique.

**9.** Emulsion grasse selon l'une quelconque des revendications 1 à 6, qui est sous la forme d'une pommade ophtalmologique.

**10.** Emulsion grasse selon la revendication 9, dans laquelle la pommade comprend une poudre de l'émulsion grasse séchée par congélation et une base de pommade.

**11.** Emulsion grasse selon une quelconque revendication précédente, dans laquelle l'ingrédient actif est le flurbiprofène.

**12.** Emulsion grasse selon l'une quelconque des revendications 1 à 10, dans laquelle l'ingrédient actif est un dérivé du flurbiprofène,ce dérivé étant un ester de flurbiprofène représenté par la formule :

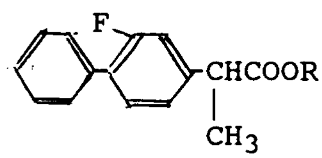

dans laquelle R est un groupe alkyle $C_1$-$C_{18}$ ou un groupe de la formule :

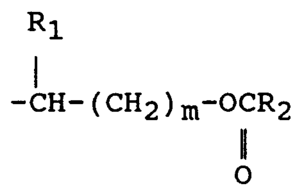

dans laquelle $R_1$ est un atome d'hydrogène ou un groupe alkyle $C_1$-$C_3$, $R_2$ est un groupe alkyle $C_1$-$C_{15}$ ou un groupe alkylényle $C_2$-$C_8$, et m vaut zéro ou 1; ou un groupe lactone de la formule :

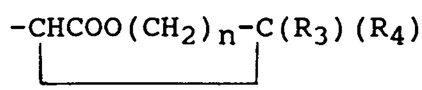

dans laquelle $R_3$ et $R_4$ sont identiques ou différents l'un de l'autre, chacun étant un atome d'hydrogène ou un groupe alkyle $C_1$-$C_3$, et n vaut 1 ou 2.

**13.** Emulsion grasse selon la revendication 12, dans laquelle le dérivé du flurbiprofène est l'ester cétylique de flurbiprofène, l'ester acétoxyméthylique de flurbinrofène: ou l'ester acétoxyéthylique de flurbiprofène.

**14.** Utilisation d'une émulsion grasse dans la fabrication d'un médicament ophtalmologique pour le traitement topique d'inflammations de l'oeil, cette émulsion grasse contenant un émulsifiant phospholipidique, une huile de soja émulsionnée et du flurbiprofène, ou un dérivé de celui-ci soluble dans l'huile de soja, dissous dans l'huile de soja.

**15.** Utilisation selon la revendication 14, dans laquelle l'émulsion grasse est comme défini dans l'une quelconque des revendications 1 à 13.

**Patentansprüche**

**1.** Fettemulsion, die einen Phospholipidemulgator und emulgiertes Sojabohnenöl enthält, in dem als aktiver Bestandteil Flurbiprofen oder ein Derivat davon gelöst ist, wobei das Derivat ein in Sojabohnenöl löslicher Ester von Flurbiprofen ist, dadurch gekennzeichnet, daß die Fettemulsion nur für örtliche und nicht für orale Verabreichung als Arzneimittelzubereitung zur opthalmologischen Anwendung angepaßt ist.

**2.** Fettemulsion nach Anspruch 1, wobei das Fett in einem wäßrigen Medium emulgiert ist.

**3.** Fettemulsion nach Anspruch 2, die 0,001 - 5 % (Gew./Vol.) Flurbiprofen, 1 - 20 % (Gew./Vol).) Sojabohnenöl, 1 - 50 Gew.-Teile eines Phospholipids bezogen auf 100 Gew.-Teile des Sojabohnenöls und als Rest ein wäßriges Medium enthält.

**4.** Fettemulsion nach einem der vorstehenden Ansprüche, die zusätzlich eine Fettsäure mit 6 bis 20 Kohlenstoffatomen oder ein physiologisch verträgliches Salz davon als Emulgationshilfsmittel enthält.

**5.** Fettemulsion nach einem der vorstehenden Ansprüche, die zusätzlich Cholesterin, Phosphatidsäure oder ein Gemisch davon als Stabilisator enthält.

**6.** Fettemulsion nach einem der vorstehenden Ansprüche, die zusätzlich ein Albumin, ein Dextran, ein Polyvinylpyrrolidon, ein Polyalkylenglykol mit einem Molekulargewicht von 1000 bis 10 000, ein Polyoxyethylen-Polyoxypropylen-Copolymerisat mit einem Molekulargewicht von 1000 bis 20 000, Gelatine oder Hydroxyethylstärke als Stabilisator enthält.

**7.** Fettemulsion nach einem der vorstehenden Ansprüche, die gefriergetrocknet ist.

**8.** Fettemulsion nach einem der vorstehenden Ansprüche, die in Form einer Lotion zur ophthalmologischen Anwendung vorliegt.

**9.** Fettemulsion nach einem der Ansprüche 1 bis 6, die in Form einer Salbe zur ophthalmologischen Anwendung vorliegt.

**10.** Fettemulsion nach Anspruch 9, wobei die Salbe ein gefriergetrocknetes Pulver der Fettemulsion und eine Salbenbasis umfaßt.

**11.** Fettemulsion nach einem der vorstehenden Ansprüche, wobei der aktive Bestandteile Flurbiprofen ist.

**12.** Fettemulsion nach einem der Ansprüche 1 bis 10, wobei der aktive Bestandteil ein Derivat von Fluorbiprofen ist, welches Derivat ein Ester von Fluorbiprofen dargestellt durch die Formel

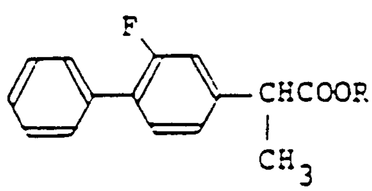

ist, wobei R ein $C_1$-$C_{18}$-Alkylrest oder ein Rest der Formel

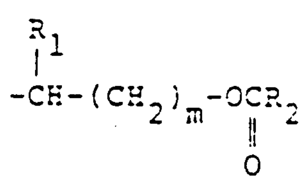

ist, wobei $R_1$ ein Wasserstoffatom oder ein $C_1$-$C_3$-Alkylrest, $R_2$ ein $C_1$-$C_{15}$-Alkylrest oder ein $C_2$-$C_8$-Alkenylrest ist, und m den Wert 0 oder 1 aufweist; oder eine Lactongruppe der Formel

$$-CHCOO(CH_2)_n-C(R_3)(R_4)$$

ist, wobei $R_3$ und $R_4$ gleich oder verschieden sind und jeweils ein Wasserstoffatom oder einen $C_1$-$C_3$-Alkylrest bedeuten und n den Wert 1 oder 2 aufweist.

**13.** Fettemulsion nach Anspruch 12, wobei das Flurbiprofenderivat Flurbiprofencetylester, Flurbiprofenacetoxymethylester oder Flurbiprofenacetoxyethylester ist.

**14.** Verwendung einer Fettemulsion bei der Herstellung eines Medikaments zur ophthalmologischen Anwendung zur örtlichen Behandlung von Entzündungen des Auges, wobei die Fettemulsion einen Phospholipidemulgator, ein emulgiertes Sojabohnenöl und Flurbiprofen oder ein in Sojabohnenöl lösliches Derivat davon, das in Sojabohnenöl gelöst ist, enthält.

**15.** Verwendung nach Anspruch 14, wobei die Fettemulsion wie in einem der Ansprüche 1 bis 13 definiert ist.